# EUROPEAN PATENT SPECIFICATION

(11) **EP 0 643 721 B1**
(45) Date of publication and mention of the grant of the patent: **16.05.2001**
(21) Application number: 92918677.3
(22) Date of filing: 14.02.1991
(51) Int. Cl.: C07H 21/02, C07H 21/04

(54) **METHOD OF SYNTHESIZING SULFURIZED OLIGONUCLEOTIDE ANALOGS**
VERFAHREN ZUR HERSTELLUNG VON SULFIERTE OLIGONUKLEOTID-ANALOGE
PROCEDE PERMETTANT DE SYNTHETISER DES ANALOGUES D'OLIGONUCLEOTIDES SULFURES

(43) Date of publication of application: 22.03.1995
(73) Proprietor: PE Corporation (NY), Foster City California 94404 (US)
(72) Inventor: HIRSCHBEIN, Bernard, L., San Francisco, CA 94110 (US)
(74) Representative: West, Alan Harry
(86) International application number: US9101008
(87) International publication number: WO9214742

(56) References cited:
- EP-A- 0 360 609
- JOURNAL OF ORGANIC CHEMISTRY, vol.55, 1990, EASTON US pages 4693 - 4699 IYER R.P. ET AL 'The Automated Synthesis of Sulfur-Containing Oligodeoxribonucleotides Using 3H-1,2-Benozodithiol-3-one 1,1-Dioxide as a Sulfur-Transfer Reagent'
- Tetrahedron Letters, Vol. 30, No. 48, issued 1989, KAMER et al., "An Efficient Approach Toward the Synthesis of Phosphorothioate Diesters via the Schoenberg Reaction", pages 6757-6760, see the entire article.
- Tetrahedron Letters, Vol. 31, No. 6, issued 1990, PETERSEN et al., "Chemical Synthesis of Dimer Ribonucleotides Containing Internucleotidic Phosphorodithioate Linkages", pages 911-914, see the entire article.
- Tetrahedron Letters, Vol. 31, No. 49, issued 1990, MORVAN et al., "Modified Oligonucleotides: IV1 Solid Phase Synthesis and Preliminary Evaluation of Phosphorothioate RNA as Potential Antisense Agents", pages 7149-7152, see the entire article.
- Nucleic Acid Research, Vol. 18, No. 4, issued 1990, "Synthesis and Properties of Dithymidine Phosphate Analogues Containing 3'-thiothymidine", pages 829-835, see the entire article.

## Description

The invention relates generally to the synthesis of oligonucleotides, and more particularly, to a method for sulfurizing oligonucleotides with thiuram disulfides to form phosphorothioate and/or phosphorodithioate analogs thereof.

With the development of efficient methods of synthesis, interest has arisen in the use of anti-sense oligonucleotides to treat a variety of diseases, particularly viral infections, e.g. Matsukura et al, Proc. Natl. Acad. Sci., Vol. 86, pgs. 4244-4448 (1989). An antisense oligonucleotide is a synthetic oligonucleotide of varying length, usually in the range of about 12 to 30 nucleotides, or nucleotide analogs, whose sequence is complementary to a predetermined segment of the RNA, either freshly transcribed or the messenger (mRNA), critical to some viral function. It is believed that when an antisense oligonucleotide hybridizes to its target RNA, it either blocks translation or processing of the RNA or makes it susceptible to enzymatic degradation.

One problem with this approach has been the difficulty of getting the antisense oligonucleotide to its target RNA in sufficient concentration and for sufficient duration to be effective in shutting down the synthesis of undesired proteins, e.g. viral enzymes, coat proteins, and the like. The susceptibility of the phosphodiester linkage of the oligonucleotides to nuclease digestion is believed to be an important cause of this difficulty, and has prompted the development of a variety of nucleoside oligomers linked by nuclease-resistant analogs of the natural phosphodiester bond, e.g. Miller et al, U.S. patent 4,511,713 and Ts'o U.S. patent 4,469,863 (methyl- and arylphosphonates); Miro et al, Nucleic Acids Research, Vol. 17, pgs. 8207-8219 (1989) (phosphoroselenoates); Brill et al, J. Am. Chem. Soc., Vol. 111, pg. 2321 (1989) (phosphorodithioates); and Matsukura et al, Proc. Natl. Acad. Sci., Vol. 84, pgs. 7706-7710 (1987), and Gene, Vol. 72, pgs. 343-347 (1988) (phosphorothioates).

The phosphorothioate and phosphorodithioate analogs are especially promising because they are highly nuclease-resistant, have the same charge as natural oligonucleotides, and are taken up by cells in effective amounts.

Phosphorothioates are conveniently synthesized by automated DNA synthesizers using hydrogen phosphonate chemistry, which permits the phosphonate backbone to be sulfurized in a single step off of the automated synthesizer after synthesis. This is advantageous because the phosphonate moieties are sulfurized by exposure to elemental sulfur dissolved in an organic solvent. Since the sulfur readily precipitates out of solution, the off-column sulfurization avoids costly blockages of valves and tubing of the synthesizer by sulfur precipitates. A drawback of of this route of phosphorothioate synthesis is that coupling yields during chain elongation are typically lower than those obtained using phosphoramidite chemistry, Gaffney and Jones, Tett. Lett., Vol. 29, pgs. 2619-2622 (1988). The practical importance of high coupling yields is demonstrated by the synthesis of a 28-mer where a 99% coupling yield per step results in an overall yield of 76% (.99²⁷), whereas a 96% yield per step results in an overall yield of only 33% (.96²⁷).

Phosphoramidite chemistry, with coupling yields typically greater than 99%, would be a highly desirable approach to phosphorothioate and phosphorodithioate synthesis. However, the phosphite intermediates, which would be sulfurized, are unstable under the conditions of the detritylization step of the reaction cycle. This requires that the phosphite linkage be sulfurized after each coupling step. For practical purposes, such sulfurizations would have to be carried out on an automated synthesizer, but the sulfur precipitation problem discussed above precludes the use of any of the commercially available machines. Moreover, the sulfurization rate of the phosphites is relatively slow and suffers from side reactions that lead to increased contamination of the final product.

Kamer et al Tetrahedron Letters, Vol. 30, pp 6757-6760 (1989) have described the uses of phenacetyl and benzyl disulphide for the rapid P-sulfurization of phosphate triesters and H-phosphonate diesters, respectively, but such reactions involve dimer yields of about 90% only. Other recent methods for synthesizing dimeric species with phosphoro(di)thioate linkages have been described by Peterson et al., ibid, Vol. 31. pp 911-914 (1990); by Morvan et al., ibid, Vol. 31, pp 7149-7152 (1990); in EP-A-0306609; and by Radhakrishnan et al., J. Org. Chem. 1990, 55, pp 4693-4699.

In view of the desire to employ phosphorothioate and phosphorodithioate analogs of oligonucleotides as pharmaceutical compounds, it would be advantageous to have available a method for sulfurizing that achieved the highest possible yields of completely sulfurized analogs and that was amenable for use with automated synthesizers, particularly with phosphoramidite and/or phosphorthioamidite chemistries.

In accordance with the invention, there is provided a method of sulfurizing a phosphorous (III)-containing compound comprising the step of reacting the phosphorous (III)-containing compound with a thiuram disulfide selected from the group defined by the formula: 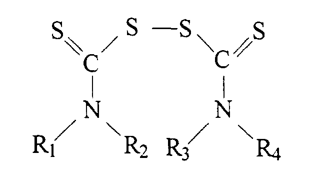 wherein:
R₁, R₂, R₃ and R₄ taken separately, are:
   hydrogen;
   alkyl having from 1 to 6 carbon atoms;
   halo-, nitro-, or cyano-substituted alkyl having from 1 to 6 carbon atoms;
   or a heterocycle containing from 5 to 8 carbon atoms and a heteroatom selected from nitrogen, oxygen, and sulfur;
R₁ and R₂ when taken together are:
   cycloalkyl having from 4 to 7 carbon atoms;
   a heterocycle containing from 3 to 6 carbon atoms and a heteroatom selected from nitrogen, oxygen, and sulfur;
R₃ and R₄ when taken together are:
   cycloalkyl having from 4 to 7 carbon atoms;
   or a heterocycle containing from 3 to 6 carbon atoms and a heteroatom selected from nitrogen, oxygen, and sulfur; and
R₂ and R₃ when taken together are a bond so that the thiuram disulfide is a substituted 1,2-dithio-4,5-diaza heterocycle.

In accordance with another aspect of the invention, there is provided a method of synthesizing a sulfurized oligonucleotide analog of a predetermined sequence comprising sulfurizing a phosphorous (III) linkage with a thiuram disulfide of the above formula I. Preferably, this method comprises the steps of:
(a) providing a protected nucleoside or analog thereof attached to a solid phase support, the protected nucleoside or analog thereof having a blocked functionality;
(b) deblocking the blocked functionality to form a reactive functionality;
(c) reacting with the reactive functionality a blocked protected nucleoside phosphoramidite or phosphorthioamidite monomer or analog thereof to form a correct-sequence chain having a phosphorous (III) linkage and a blocked functionality;
(d) sulfurizing the phosphorous (III) linkage by exposing the correct-sequence chain to a thiuram disulfide; and
(e) repeating steps (b) through (d) until the sulfurized oligonucleotide of the predetermined sequence is obtained.

Preferably, in the formula I, R₁, R₂, R₃, and R₄, taken separately, are methyl, ethyl, propyl, isopropyl, n-butyl, sec-butyl, tert-butyl, n-pentyl, cyclopentylmethyl, isopentyl, neopentyl, n-hexyl, neohexyl, isohexyl, cyclohexylmethyl, beta-cyclopentylethyl, nitro, lower alkyl-, nitro-, or halo-substituted phenyl, lower alkyl- or halo-substituted benzyl, or lower alkyl-, nitro-, or halo-substituted phenylethyl, morpholinyl, thiomorpholinyl, piperidinyl, piperazinyl, beta-electron-withdrawing-substituted ethyl, or the like. In further preference, the electron-withdrawing substituent of beta-electron-withdrawing-substituted ethyl is cyano, nitro, phenylsulphonyl, or phenylester. Most preferably, the beta-electron-withdrawing-substituted ethyl is betacyanoethyl. In further preference, the lower alkyl-, nitro-, or halo-substituents of the lower alkyl-, nitro-, or halo-substituted phenyl and benzyl are methyl, chloro, or bromo. In further preference, morpholinyl, thiomorpholinyl, and piperidinyl are morpholino, thiomorpholino, and piperidino, respectively. Most preferably, R₁, R₂, R₃, and R₄, taken separately, are methyl, ethyl, or isopropyl.

R₁ and R₂ when taken together are cycloalkyl having from 4 to 7 carbon atoms or a heterocycle containing nitrogen, oxygen, or sulfur and from 3 to 6 carbon atoms; more preferably, when taken together, R₁ and R₂ are cycloalkyl having 4 carbon atoms.

R₃ and R₄ when taken together are cycloalkyl having from 4 to 7 carbon atoms or a heterocycle containing nitrogen, oxygen, or sulfur and from 3 to 6 carbon atoms; more preferably, when taken together, R₃ and R₄ are cycloalkyl having 4 carbon atoms.

R₂ and R₃ when taken together are a bond so that the compound of Formula I is a substituted 1,2-dithio-4,5-diaza heterocycle.

The term "lower alkyl" as used herein denotes straight-chain and branched-chain alkyl groups containing from 1-6 carbon atoms, e.g. methyl, ethyl, propyl, isopropyl, tert-butyl, isobutyl, sec-butyl, neopentyl, tert-pentyl, and the like. "Electron-withdrawing" denotes the tendency of a substituent to attract valence electrons of the molecule of which it is apart, i.e. it is electronegative, March, Advanced Organic Chemistry, pgs. 16-18 (John Wiley, New York, 1985).

The term "oligonucleotide" as used herein includes linear oligomers of natural or modified nucleosides or of non-nucleosidic analogs linked by phosphodiester bonds or analogs thereof ranging in size from a few monomeric units, e.g. 2-3, to several hundred monomeric units. In particular, the term includes non-natural oligomers having phosphorous-containing linkages whose phosphorous(III) precursors are amenable to sulfurization, e.g. Takeshita et al, J. Biol. Chem., Vo. 282, pgs. 10171-10179 (1987); and Eapienis et al, pgs. 225-230 in, Bruzik and Stec, eds., Biophosphates and Their Analogs--Synthesis, Structure, Metabolism, and Activity (Elsevier, Amsterdam, 1986).

The invention includes a method of synthesizing phosphorothioates and phosphorodithioates. An important feature of the invention is the step of reacting phosphorous III-containing moieties of oligonucleotide intermediates with a thiuram disulfide to bring about sulfurization. Because thiuram disulfides are efficient sulfurizing agents that do not precipitate out of solution, the invention is particularly useful in the automated synthesis of phosphorothioate and phosphorodithioate analogs of oligonucleotides by all the commercially viable approaches, including hydrogen phosphonate, phosphoramidite, or phosphorothioamidite chemistries.

Detailed procedures for the phosphoramidite, phosphorthioamidite, and hydrogen phosphonate methods of oligonucleotide synthesis are described in the following references, which are incorporated by reference: Caruthers et al, U.S. Patents 4,458,066 and 4,500,707; Koester et al, U.S. patent 4,725,677; Matteucci et al, J. Amer. Chem. Soc., Vol. 103, pgs. 3185-3191 (1981); Caruthers et al, Genetic Engineering, Vol. 4, pgs. 1-17 (1981); Jones, chapter 2, and Atkinson et al, chapter 3, in Gait, ed., Oligonucleotide Synthesis: A Practical Approach (IRL Press, Washington, D.C., 1984); Froehler et al, Tetrahedron Letters, Vol. 27, Pgs. 469-472 (1986); Garegg et al, Tetrahedron Letters, Vol. 27, pgs. 4051-4054 and 4055-4058 (1986); Andrus et al, U.S. patent 4,816,571; Brill et al, J. Am. Chem. Soc., Vol. 111, pgs. 2321- (1989); and Froehler et al, Nucleic Acids Research, Vol. 14, pgs. 5399-5407 (1986).

Thiuram disulfides are well known compounds that have many industrial uses, including uses as fungicides, topical antiseptics, and accelerators in the synthesis of rubber. Properties and methods of synthesizing thiuram disulfides are also well known, and are described in the following references which are incorporated by reference: U.S. patent 1,782,111; U.S. patent 1,796,977; Nash et al, pgs. 168-191 in Florey, ed. Analytical Profiles of Drug Substances, Vol. 4 (Academic Press, New York, 1975); Cummings et al, Indust. Eng. Chem., Vol. 20, pgs. 1173-1176 (1928); and World Health Organization, pgs. 225-236 in IARC Monographs on the Evaluation fo Carcinogenic Risk of Chemicals to Man, Vol. 12 (1976). As indicated by these references, thiuram disulfides are synthesized in several different ways, including by the oxidation of the dimethylamine salt of dimethyldithiocarbamic acid with iodine in an ethanolic solution, e.g. von Braun, I. Chem. Ber., Vol. 35, pgs. 817-829 (1902), by passing chlorine gas through a solution of sodium dimethyldithiocarbamate, e.g. Wenyon, pgs. 621-623 in Chemical Technology: An Encyclopedic Treatment, Vol. 4 (Barnes and Noble, New York, 1972), or by oxidation of sodium dimethyldithiocarbamate with hydrogen peroxide or iodine, e.g. Spencer, Guide to the Chemical Used in Crop Protection, 6th Ed., University of Western Ontario, Publication 1093 (London, Ontario, 1973).

When employed as a sulfurizing agent in the hydrogen phosphonate approach, a thiuram disulfide is delivered to the completed oligonucleotide chain in a suitable organic solvent, such as acetonitrile, tetrahydrofuran, dichloromethane, or the like, in a consentration of between about .01 M to about 2.0 M. Preferably, the sulfurization is accomplished on an automated DNA synthesizer, e.g. an Applied Biosystems model 380B, or like machine.

Most preferably, thiuram disulfides are employed as a sulfurizing agents in the phosphoramidite or phosphorthioamidite approaches. A thiuram disulfide is delivered to the growing oligomer as a separate step within each addition cycle. Generally, the addition cycles of these methods of synthesis involve the following steps: (1) deblocking a blocked functionality (usually a 5'-tritylated hydroxyl) on the growing correct-sequence chain, or on the initial monomer attached to a solid phase support, to form a reactive functionality (e.g. a 5'-hydroxyl), (2) reacting a blocked and protected nucleoside phosphoramidite or phosphorthioamidite monomer or analog thereof (usually in the presence of an activator, e.g. tetrazole) with the reactive functionality of the growing correct-sequence chain, (3) capping unreacted reactive functionalities, and (4) oxidizing the newly formed phosphorous(III) linkage to form the naturally occurring pentacoordinate state. The sequence of above steps (3) and (4) can be reversed. The term "protected" in reference to monomer, particularly nucleoside phosphoramidites or phosphorthioamidites, means that moieties such as exocyclic nitrogens, 2'-hydroxyls, oxygens bonded to the phosphorous, or the like, have protection groups (usually base-labile) attached which are removed after synthesis is completed, e.g. as those described in Koester et al (cited above), or in Molko et al, European patent publication no. 241,363 dated 14 October 1987. The term is also meant to include monomers which may not have moieties requiring protective groups, e.g. some nucleoside analogs, abasic nucleosides, and the like. In the method of the invention, thiuram disulfides are employed as sulfurizing agents in place of the oxidation step. Preferably, a thiuram disulfide is delivered to the growing oligomer in a suitable organic solvent, such as acetonitrile, tetrahydrofuran, dichloromethane, or the like, in a concentration of between about .01 M to about 2.0 M. Preferably, the step of sulfurizing with a thiuram disulfide is accomplished on an automated DNA synthesizer. In both approaches a wide variety of reaction temperatures may be used. Preferably, the sulfurization is carried out at a temperature in the range of 0°C to 100°C, and more preferably, in the range of 15°C to 60°C.

### EXAMPLE

### Synthesis of a 22-base Phosphorothioate Oligonucleotide Using Tetraethylthiuram Disulfide as Sulfurizing Agent

A 22-base phosphorothioate oligonucleotide, 5'-CTTCGATCATCGGTATGCTCCT, was synthesized by the phosphoramidite method on an automated synthesizer, on which the reaction vessel was modified by wrapping it with a resistive heating element with integral RTD (Watlo, St. Louis, MO) to provide control of reaction temperature. The heating element was connected to an Omron temperature controller with an external relay. tape to provide control of reaction temperature. The standard synthesis protocol was followed, except that in place of the oxidation step, a sulfurization step was substituted. In other words, the synthesis consisted of repeated cycles of detritylization, coupling, sulfurization, and capping. Separation of the final product from the synthesis column and purification were accomplished by standard means. The sulfurization step was accomplished by exposing the growing chain to 0.55 M tetraethylthiuram disulfide (Aldrich, Milwaukee, WI) in acetonitrile at 50°C for 15 minutes. The tetraethylthiuram disulfide can be conveniently recrystallized from acetonitrile; however, no difference was noted in results using recrystallized material or material as supplied by the manufacturer.

The yield of trityl cation released during the detritylization steps averaged 99%. The trityl yield is a both a measure of coupling efficiency and a measure of the extent of sulfurization, since non-sulfurized (or oxidized) trivalent phosphorous linkages in the oligonucleotide are labile to cleavage during detritylization.

The 22-mer was cleaved from the support and deprotected with concentrated ammonium hydroxide at 55°C for 3 hours. The ³¹P-NMR spectra (JEOL, 36.5 MHz, ppm vs H₃PO₄ external reference) of the product showed greater than 98% sulfur incorporation (55.1 ppm) with less than 2% oxygen incorporation (-1.1 ppm).

## Claims

1. A method of sulfurizing a phosphorous (III)-containing compound comprising the step of reacting the phosphorous (III)-containing compound with a thiuram disulfide selected from the group defined by the formula: 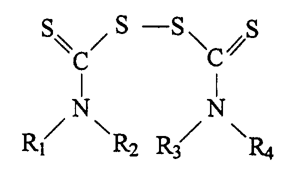 wherein:
R₁, R₂, R₃ and R₄ taken separately, are:
hydrogen;
alkyl having from 1 to 6 carbon atoms;
halo-, nitro-, or cyano-substituted alkyl having from 1 to 6 carbon atoms;
or a heterocycle containing from 5 to 8 carbon atoms and a heteroatom selected from nitrogen, oxygen, and sulfur;
R₁ and R₂ when taken together are:
cycloalkyl having from 4 to 7 carbon atoms;
a heterocycle containing from 3 to 6 carbon atoms and a heteroatom selected from nitrogen, oxygen, and sulfur;
R₃ and R₄ when taken together are:
cycloalkyl having from 4 to 7 carbon atoms;
or a heterocycle containing from 3 to 6 carbon atoms and a heteroatom selected from nitrogen, oxygen, and sulfur; and
R₂ and R₃ when taken together are a bond so that the thiuram disulfide is a substituted 1,2-dithio-4,5-diaza heterocycle.

2. A method as claimed in claim 1 wherein the phosphorous (III)-containing compound is a linear oligomer having at least one phosphite or hydrogen phosphonate diester linkage.

3. A method as claimed in any preceding claim wherein:
R₁, R₂, R₃ and R₄, taken separately, are hydrogen, methyl, ethyl, propyl, isopropyl, n-butyl, sec-butyl, tert-butyl, n-pentyl, cyclopentylmethyl, isopentyl, neopentyl, n-hexyl, neohexyl, isohexyl, cyclohexylmethyl, beta-cyclopentylethyl, lower alkyl-, nitro-, or halo-substituted phenyl, lower alkyl-, nitro-, or halo-substituted benzyl, or a lower alkyl-, nitro-, or halo-substituted phenylethyl, morpholinyl, thiomorpholinyl, piperidinyl, piperazinyl;
R₁ and R₂ when taken together are cycloalkyl having 4 carbon atoms; and
R₃ and R₄ when taken together are cycloalkyl having 4 carbon atoms.

4. A method as claimed in claim 1 or claim 2 wherein R₁, R₂, R₃ and R₄ taken separately, are hydrogen, methyl, ethyl, or isopropyl.

5. A method of synthesizing a sulfurized oligonucleotide analog of a predetermined sequence comprising sulfurizing a phosphorous (III) linkage with a thiuram disulfide selected from the group defined by the formula 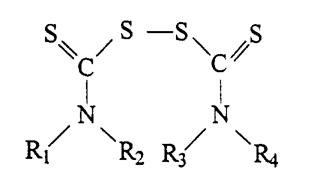 wherein:
R₁, R₂, R₃ and R₄ taken separately, are:
hydrogen;
alkyl having from 1 to 6 carbon atoms;
halo-, nitro-, or cyano-substituted alkyl having from 1 to 6 carbon atoms;
or a heterocycle containing from 5 to 8 carbon atoms and a heteroatom selected from nitrogen, oxygen, and sulfur;
R₁ and R₂ when taken together are:
cycloalkyl having from 4 to 7 carbon atoms;
a heterocycle containing from 3 to 6 carbon atoms and a heteroatom selected from nitrogen, oxygen, and sulfur;
R₃ and R₄ when taken together are:
cycloalkyl having from 4 to 7 carbon atoms;
or a heterocycle containing from 3 to 6 carbon atoms and a heteroatom selected from nitrogen, oxygen, and sulfur; and
R₂ and R₃ when taken together are a bond so that the thiuram disulfide is a substituted 1,2-dithio-4,5-diaza heterocycle.

6. A method as claimed in claim 5 comprising the steps of:
(a) providing a protected nucleoside or analog thereof attached to a solid phase support, the protected nucleoside or analog thereof having a blocked functionality;
(b) deblocking the blocked functionality to form a reactive functionality;
(c) reacting with the reactive functionality a blocked protected nucleoside phosphoramidite or phosphorthioamidite monomer or analog thereof to form a correct-sequence chain having a phosphorous (III) linkage and a blocked functionality;
(d) sulfurizing the phosphorous (III) linkage by exposing the correct-sequence chain to a thiuram disulfide; and
(e) repeating steps (b) through (d) until the sulfurized oligonucleotide of the predetermined sequence is obtained.

7. A method as claimed in claim 6 further comprising the step of capping unreacted reactive functionalities after said step of sulfurizing.

8. A method as claimed in claim 6 or claim 7 further comprising the step of cleaving said sulfurized oligonucleotide analog from said solid phase support.

9. A method as claimed in any one of claims 6, 7 or 8 wherein said blocked functionality is a tritylated 5'-hydroxyl of the correct-sequence chain or of the protected nuceloside or an analog thereof.

10. A method as claimed in any one of claims 6, 7, 8 or 9 wherein:
R₁, R₂, R₃ and R₄, taken separately, are methyl, ethyl, propyl, isopropyl, n-butyl, sec-butyl, tert-butyl, n-pentyl, cyclopentylmethyl, isopentyl, neopentyl, n-hexyl, neohexyl, isohexyl, cyclohexylmethyl, beta-cyclopentylethyl, lower alkyl-, nitro-, or halo-substituted phenyl, lower alkyl-, nitro-, or halo-substituted benzyl, or a lower alkyl-, nitro-, or halo-substituted phenylethyl, morpholinyl, thiomorpholinyl, piperidinyl, piperazinyl;
R1 and R2 when taken together are cycloalkyl having 4 carbon atoms; and
R3 and R4 when taken together are cycloalkyl having 4 carbon atoms.

11. The method of claim 11 wherein R₁, R₂, R₃ and R₄, taken separately are hydrogen, methyl, ethyl, or isopropyl.

## Patentansprüche

1. Verfahren zum Schwefeln einer Phosphor(III) enthaltenden Verbindung, das den Schritt umfaßt, die Phosphor(III) enthaltende Verbindung mit einem Thiuramdisulfid zur Reaktion zu bringen, das aus der durch die folgende Formel definierten Gruppe ausgewählt ist: 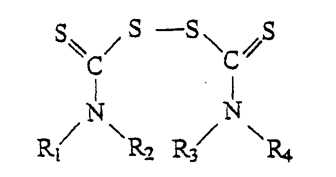 wobei:
R₁, R₂, R₃ und R₄ getrennt genommen:
Wasserstoff;
Alkyl mit von 1 bis 6 Kohlenstoffatomen;
Halogen-, Nitro- oder Cyano-substituiertes Alkyl mit von 1 bis 6 Kohlenstoffatomen;
oder einen von 5 bis 8 Kohlenstoffatome enthaltenden Heterocyclus und ein aus Stickstoff, Sauerstoff und Schwefel ausgewähltes Heteroatom darstellen;
R₁ und R₂, wenn zusammen genommen:
Cycloalkyl mit von 4 bis 7 Kohlenstoffatomen;
einen von 3 bis 6 Kohlenstoffatomen enthaltenden Heterocyclus und ein aus Stickstoff, Sauerstoff und Schwefel ausgewähltes Heteroatom darstellen;
R₃ und R₄, wenn zusammen genommen:
Cycloalkyl mit von 4 bis 7 Kohlenstoffatomen;
oder einen von 3 bis 6 Kohlenstoffatomen enthaltenden Heterocyclus und ein aus Stickstoff, Sauerstoff und Schwefel ausgewähltes Heteroatom darstellen; und
R₂ und R₃, wenn zusammen genommen, eine Bindung sind, so daß das Thiuramdisulfid ein substituierter 1,2-Dithio-4,5-Diaza-Heterocyclus ist.

2. Verfahren nach Anspruch 1, bei dem die Phosphor(III) enthaltende Verbindung ein lineares Oligomer mit wenigstens einer Phosphit- oder Wasserstoffphosphonatdiesterbindung darstellt.

3. Verfahren nach einem vorhergehenden Anspruch, bei dem:
R₁, R₂, R₃ und R₄ getrennt genommen Wasserstoff, Methyl, Ethyl, Propyl, Isopropyl, n-Butyl, sec-Butyl, tert-Butyl, n-Pentyl, Cyclopentylmethyl, Isopentyl, Neopentyl, n-Hexyl, Neohexyl, Isohexyl, Cyclohexylmethyl, beta-cyclopentylethyl, niederes Alkyl-, Nitro- oder Halogen-substituiertes Phenyl, niederes Alkyl-, Nitro- oder Halogen-substituiertes Benzyl, oder ein niederes Alkyl-, Nitro-, oder Halogen-substituiertes Phenylethyl, Morpholinyl, Thiomorpholinyl, Piperidinyl, Piperazinyl darstellen;
R₁ und R₂, wenn zusammen genommen, Cycloalkyl mit 4 Kohlenstoffatomen sind; und
R₃ und R₄, wenn zusammen genommen, Cycloalkyl mit 4 Kohlenstoffatomen darstellen.

4. Verfahren nach Anspruch 1 oder Anspruch 2, bei dem R₁, R₂, R₃ und R₄ getrennt genommen Wasserstoff, Methyl, Ethyl oder Isopropyl darstellen.

5. Verfahren zum Synthetisieren einer geschwefelten Oligonukleotidentsprechung einer vorbestimmten Sequenz, das umfaßt, eine Phosphor(III)-Bindung mit einem Thiuramdisulfid ausgewählt aus der durch die folgende Formel definierten Gruppe zu schwefeln: 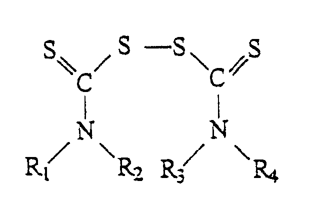 wobei:
R₁, R₂, R₃ und R₄ getrennt genommen:
Wasserstoff;
Alkyl mit von 1 bis 6 Kohlenstoffatomen;
Halogen-, Nitro- oder Cyano-substituiertes Alkyl mit von 1 bis 6 Kohlenstoffatomen;
oder einen von 5 bis 8 Kohlenstoffatome enthaltenden Heterocyclus und ein aus Stickstoff, Sauerstoff und Schwefel ausgewähltes Heteroatom darstellen;
R₁ und R₂, wenn zusammen genommen:
Cycloalkyl mit von 4 bis 7 Kohlenstoffatomen;
einen von 3 bis 6 Kohlenstoffatomen enthaltenden Heterocyclus und ein aus Stickstoff, Sauerstoff und Schwefel ausgewähltes Heteroatom darstellen;
R₃ und R₄, wenn zusammen genommen:
Cycloalkyl mit von 4 bis 7 Kohlenstoffatomen;
oder einen von 3 bis 6 Kohlenstoffatomen enthaltenden Heterocyclus und ein aus Stickstoff, Sauerstoff und Schwefel ausgewähltes Heteroatom darstellen; und
R₂ und R₃, wenn zusammen genommen, eine Bindung sind, so daß das Thiuramdisulfid einen substituierten 1,2-Dithio-4,5-Diaza-Heterocyclus darstellen.

6. Verfahren nach Anspruch 5, das die Schritte umfaßt:
(a) ein geschütztes Nucleosid oder eine Entsprechung desselben befestigt an einem Festphasenträger vorzusehen, wobei das geschützte Nucleosid oder die Entsprechung desselben eine blockierte Funktionalität aufweist;
(b) die Blockierung der blockierten Funktionalität zum Bilden einer reaktionsbereiten Funktionalität aufzuheben;
(c) mit der reaktionsbereiten Funktionalität ein blockiertes geschütztes Nucleosidphosphoramidit- oder Phosphorthioamiditmonomer oder eine Entsprechung desselben zur Reaktion zu bringen, um eine Kette richtiger Sequenz mit einer Phosphor(III)-Bindung und einer blockierten Funktionalität zu bilden;
(d) die Phosphor(III)-Bindung zu schwefeln, indem die Kette richtiger Sequenz einem Thiuramdisulfid ausgesetzt wird; und
(e) die Schritte (b) bis (d) zu wiederholen, bis das geschwefelte Oligonukleotid der vorbestimmten Sequenz erhalten wird.

7. Verfahren nach Anspruch 6, das weiter den Schritt umfaßt, nichtreagierte reaktionsbereite Funktionalitäten nach dem Schwefelungsschritt abzudecken.

8. Verfahren nach Anspruch 6 oder Anspruch 7, das weiter den Schritt umfaßt, die geschwefelte Oligonukleotidentsprechung von dem Festphasenträger abzuspalten.

9. Verfahren nach einem der Ansprüche 6, 7 oder 8, bei dem die blockierte Funktionalität ein trityliertes 5'-Hydroxyl der Kette richtiger Sequenz oder des geschützten Nukleosids oder einer Entsprechung desselben darstellt.

10. Verfahren nach einem der Ansprüche 6, 7, 8 oder 9, bei dem:
R₁, R₂, R₃ und R₄, getrennt genommen, Methyl, Ethyl, Propyl, Isopropyl, n-Butyl, sec-Butyl, tert-Butyl, n-Pentyl, Cyclopentylmethyl, Isopentyl, Neopentyl, n-Hexyl, Neohexyl, Isohexyl, Cyclohexylmethyl, beta-cyclopentylethyl, niederes Alkyl-, Nitro- oder Halogen-substituiertes Phenyl, niederes Alkyl-, Nitro- oder Halogen-substituiertes Benzyl, oder ein niederes Alkyl-, Nitro-, oder Halogen-substituiertes Phenylethyl, Morpholinyl, Thiomorpholinyl, Piperidinyl, Piperazinyl darstellen;
R₁ und R₂, wenn zusammen genommen, Cycloalkyl mit 4 Kohlenstoffatomen sind; und
R₃ und R₄, wenn zusammen genommen, Cycloalkyl mit 4 Kohlenstoffatomen darstellen.

11. Verfahren von Anspruch 11, bei dem R₁, R₂, R₃ und R₄, getrennt genommen, Wasserstoff, Methyl, Ethyl oder Isopropyl darstellen.

## Revendications

1. Procédé de sulfuration d'un composé contenant du phosphore (III), qui comprend l'étape de réaction du composé contenant du phosphore (III) avec un disulfure de thiurame choisi dans le groupe défini par la formule : 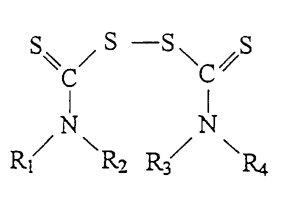 dans laquelle :
R₁, R₂, R₃ et R₄, pris séparément, représentent chacun :
un atome d'hydrogène ;
un groupe alkyle ayant de 1 à 6 atomes de carbone ;
un groupe alkyle à substitution halogéno, nitro ou cyano, ayant de 1 à 6 atomes de carbone ;
ou un groupe hétérocyclique contenant de 5 à 8 atomes de carbone et un hétéroatome choisi parmi l'azote, l'oxygène et le soufre ;
R₁ et R₂, quand ils sont pris ensemble, représentent :
un groupe cycloalkyle ayant de 4 à 7 atomes de carbone ;
un groupe hétérocyclique contenant de 3 à 6 atomes de carbone et un hétéroatome choisi parmi l'azote, l'oxygène et le soufre ;
R₃ et R₄, quand ils sont pris ensemble, représentent :
un groupe cycloalkyle ayant de 4 à 7 atomes de carbone ;
ou un groupe hétérocyclique contenant de 3 à 6 atomes de carbone et un hétéroatome choisi parmi l'azote, l'oxygène et le soufre ; et
R₂ et R₃, quand ils sont pris ensemble, représentent une liaison de telle sorte que le disulfure de thiurame soit un composé 1,2-dithio-4,5-diazahétérocyclique substitué.

2. Procédé selon la revendication 1, dans lequel le composé contenant du phosphore (III) est un oligomère linéaire ayant au moins une liaison diester hydrogénophosphonate ou phosphite.

3. Procédé selon l'une quelconque des revendications précédentes, dans lequel :
R₁, R₂, R₃ et R₄, pris séparément, représentent chacun un atome d'hydrogène ou un groupe méthyle, éthyle, propyle, isopropyle, n-butyle, sec-butyle, tert-butyle, n-pentyle, cyclopentylméthyle, isopentyle, néopentyle, n-hexyle, néohexyle, isohexyle, cyclohexylméthyle, béta-cyclopentyléthyle, phényle à substitution alkyle inférieur, nitro ou halogéno, benzyle à substitution alkyle inférieur, nitro ou halogéno, ou phényléthyle à substitution alkyle inférieur, nitro ou halogéno, morpholinyle, thiomorpholinyle, pipéridinyle, pipérazinyle ;
R₁ et R₂, quand ils sont pris ensemble, forment un groupe cycloalkyle ayant 4 atomes de carbone ; et
R₃ et R₄, quand ils sont pris ensemble, forment un groupe cycloalkyle ayant 4 atomes de carbone.

4. Procédé selon la revendication 1 ou 2, dans lequel R₁, R₂, R₃ et R₄, pris séparément, représentent chacun un atome d'hydrogène ou un groupe méthyle, éthyle ou isopropyle.

5. Procédé de synthèse d'un analogue d'oligonucléotide sulfuré ayant une séquence prédéterminée, qui comprend la sulfuration d'une liaison à phosphore (III) avec un disulfure de thiurame choisi dans le groupe défini par la formule 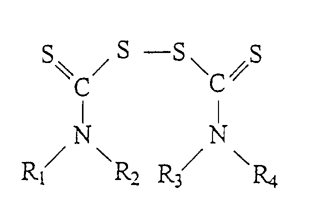 dans laquelle :
R₁, R₂, R₃ et R₄, pris séparément, représentent chacun :
un atome d'hydrogène ;
un groupe alkyle ayant de 1 à 6 atomes de carbone ;
un groupe alkyle à substitution halogéno, nitro ou cyano, ayant de 1 à 6 atomes de carbone ;
ou un groupe hétérocyclique contenant de 5 à 8 atomes de carbone et un hétéroatome choisi parmi l'azote, l'oxygène et le soufre ;
R₁ et R₂, quand ils sont pris ensemble, représentent :
un groupe cycloalkyle contenant de 4 à 7 atomes de carbone ;
un groupe hétérocyclique contenant de 3 à 6 atomes de carbone et un hétéroatome choisi parmi l'azote, l'oxygène et le soufre ;
R₃ et R₄, quand ils sont pris ensemble, représentent :
un groupe cycloalkyle ayant de 4 à 7 atomes de carbone ;
ou un groupe hétérocyclique ayant de 3 à 6 atomes de carbone et un hétéroatome choisi parmi l'azote, l'oxygène et le soufre ; et
R₂ et R₃, quand ils sont pris ensemble, représentent une liaison de telle sorte que le disulfure de thiurame soit un composé 1,2-dithio-4,5-diazahétérocyclique substitué.

6. Procédé selon la revendication 5, qui comprend les étapes de :
(a) mise à disposition d'un nucléoside protégé ou d'un analogue de ce dernier, fixé à un support en phase solide, le nucléoside protégé ou l'analogue de ce dernier ayant une fonctionnalité bloquée ;
(b) déblocage de la fonctionnalité bloquée pour former une fonctionnalité réactive ;
(c) réaction, avec la fonctionnalité réactive, d'un phosphoramidite ou phosphorothioamidite monomère de nucléoside protégé bloqué, ou d'un analogue de celui-ci, pour former une chaîne à séquence correcte ayant une liaison à phosphore (III) et une fonctionnalité bloquée ;
(d) sulfuration de la liaison à phosphore (III) par exposition de la chaîne à séquence correcte à un disulfure de thiurame ; et
(e) répétition des étapes (b) à (d) jusqu'à obtention de l'oligonucléotide sulfuré ayant la séquence prédéterminée.

7. Procédé selon la revendication 6, qui comprend en outre l'étape de coiffage des fonctionnalités réactives n'ayant pas réagi après ladite étape de sulfuration.

8. Procédé selon la revendication 6 ou 7, qui comprend en outre l'étape de séparation, d'avec ledit support en phase solide, dudit analogue d'oligonucléotide sulfuré.

9. Procédé selon l'une quelconque des revendications 6, 7 ou 8, dans lequel ladite fonctionnalité bloquée est un groupe 5'-hydroxyle tritylé de la chaîne à séquence correcte ou du nucléoside protégé, ou d'un analogue de ce dernier.

10. Procédé selon l'une quelconque des revendications 6, 7, 8 ou 9, dans lequel :
R₁, R₂, R₃ et R₄, pris séparément, représentent chacun un groupe méthyle, éthyle, propyle, isopropyle, n-butyle, sec-butyle, tert-butyle, n-pentyle, cyclopentylméthyle, isopentyle, néopentyle, n-hexyle, néohexyle, isohexyle, cyclohexylméthyle, béta-cyclopentyléthyle, phényle à substitution alkyle inférieur, nitro ou halogéno, benzyle à substitution alkyle inférieur, nitro ou halogéno, ou phényléthyle à substitution alkyle inférieur, nitro ou halogéno, morpholinyle, thiomorpholinyle, pipéridinyle, pipérazinyle ;
R₁ et R₂, quand ils sont pris ensemble, forment un groupe cycloalkyle ayant 4 atomes de carbone ; et
R₃ et R₄, quand ils sont pris ensemble, forment un groupe cycloalkyle ayant 4 atomes de carbone.

11. Procédé selon la revendication 10, dans lequel R₁, R₂, R₃ et R₄, pris séparément, représentent chacun un atome d'hydrogène ou un groupe méthyle, éthyle ou isopropyle.
